Europäisches Patentamt

European Patent Office

Office européen des brevets

(11) Publication number: **0 428 815 A1**

# EUROPEAN PATENT APPLICATION

(21) Application number: 89830521.4

(22) Date of filing: 24.11.89

(51) Int. Cl.5: **C07G 17/00, A61K 35/78**

(43) Date of publication of application:
29.05.91 Bulletin 91/22

(84) Designated Contracting States:
AT BE CH DE ES FR GB GR IT LI LU NL SE

(71) Applicant: SEARCH BIOLOGICAL
TECHNOLOGY COMPANY
9th Floor-1, No. 188, Chien Kuo North Road,
Sec. 2

Taipei(TW)

(72) Inventor: **Chia Chen, Chen**
**9th Floor-1 No. 188**
**Chien Kuo North Road, Sec. 2 Taipei(TW)**

(74) Representative: **Righetti, Giuseppe**
**Bugnion S.p.A. Via Carlo Farini, 81**
**I-20159 Milano(IT)**

(54) Process of purifying a cytolytic toxin from plant Garcinia morella Desv. (G.M.D.) resin for anticancer study.

(57) An active component with high chemical stability was identified and purified from resin of plant G.M.D. The resulting product has been extensively studied for its pharmacological and toxicological activities. Data indicated that the active component functions as a very potent cytolytic chemical compound. Cells were irreversibly killed at very low concentration less than 10ng per ml. With such low concentration, there was however, very little evidence of intoxication. In this report, we have invented various rapid methods for extracting the active component GMD 1630 in large quantity and way of long term storage. Also, in vitro bioassays and in vivo tests for monitoring intoxication were designed.

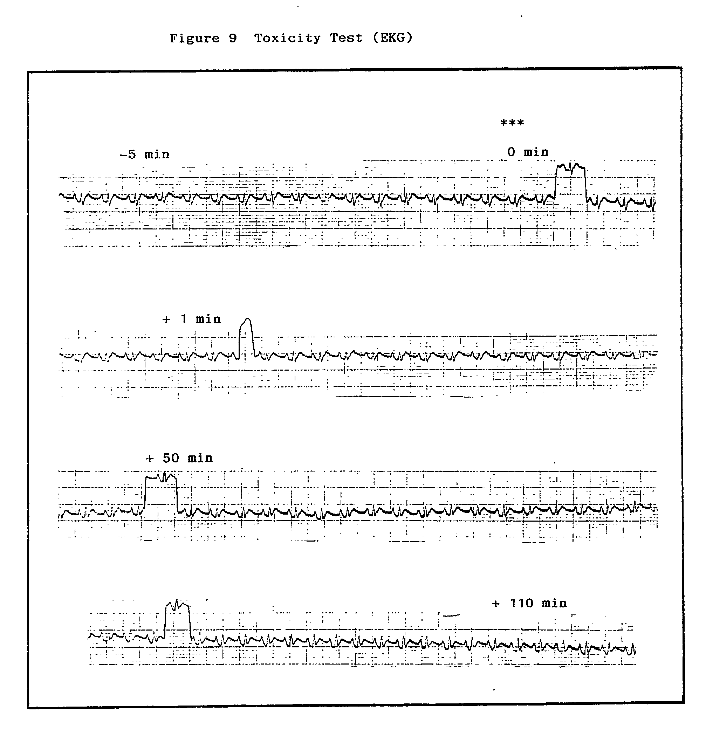

Figure 9  Toxicity Test (EKG)

# PROCESS OF PURIFYING A CYTOLYTIC TOXIN FROM PLANT GARCINIA MORELLA DESV. (G.M.D.) RESIN FOR ANTICANCER STUDY

The invention relates to the field of purifying material useful for cytolytic study and potential application for anticancer treatment. More precisely, the invention relates to a new potent cytotoxic agent prepared from a series of extraction procedures. The purification has yield a suitable product for mechanism investigation and potential clinical trial.

A great variety of compounds has been investigated in experimental animals and a few have been proven sufficiently useful in the clinical treatment of human neoplasms, at acceptable levels of toxicity, to deserve the designation of chemotherapeutic agents. Many classes of antineoplastic drugs have been well studied. These include alkylating agents, antimetabolites, natural products, drugs of miscellaneous agents, hormones and antagonists, and radioactive isotopes.

Understanding the common events for tumor formation but also the heterogeneity of tumor has generated the discovery of many anticancer drugs. Thirty years ago, significant palliative results were achieved with chemotherapy for a number of human neoplasms, and the first indications had emerged that choriocarcinoma in women could be cured by treatment with methotrexate. Today, a substantial number of neoplastic diseases were found that need not shorten life if treated with drugs or drugs with other modalities. These include choriocarcinoma in women; acute leukemia, Wilm's tumor, Ewing's sarcoma, rhabdomyosarcoma and retinoblastoma in children; and Hodgkin's desease, diffuse histiocytic lymphoma, Burkitt's lymphoma, mycosis fungoides and testicular carcinoma. Despite these impressive results, there is realization that many of the most common forms of human cancer still resist effective chemotherapeutic treatment.

Fundamental information related to the basic mechanism for tumor behavior continue to be made. Efforts for discovering new useful chemotherapeutic agents and designing more effective regimens plus less toxicity has gained greatest progress in the last ten years.

There are many ancient reports related to the application of Chinese herb material for "Mass Resolution" purpose. The term "mass resolution" was not precise at all, while mass may be due to inflammation, tumor mass or some other causes. It is important to classify the targeted deseases in modern medical term. More usefully will it be for research and application if we may even purify the active component from the crude herb material.

In China, G.M.D. plant resin had been reported as an antibiotics-like substance for treating carbuncles. Many compounds include morellin, morellic acid, morell of lavone, alfa-guttiferin and beta-guttiferin were isolated from its extract. Many was reported to have the anti-staphylococcus effect. However, little was known about its toxicity and no information available for the described cytolytic effect in this report.

For designing a convenient and sensitive detection method, a cell line out of many was selected. This cell line CE with rapid growth rate provides an unexhausted source for mammalian cells. Also CE cells showed rapid lytic response within two hours after adding the toxin. This method along with two other assays was used for investigating the mechanism for its biological effects and monitoring intoxication during in vivo application.

The invention provides a few extraction steps for obtaining an active component GMD 1630 of cytolytic property. The extraction procedures are designed to be suitable for rapid collection of active component from G. M.D. plant resin block in high yield. During the procedure, many unwanted components were removed and purified material became sterilized by various organic solvents. The invention also described a few bioassays for detecting the presence of GMD 1630. These are essential for investigating the mechanism of intoxication and useful in monitoring the in vivo concentration.

Extraction steps have been designed in a flexible way that for purification, there are all effective optional orders of doing these different extractions. Two major approaches: one using absolute alcohol and the other using chloroform as the two solvent systems has been applied and combined to have the high yield and good purification.

For ethanol extraction system, resin block (obtained either from herb drug store or from natural sources as the dried plant extract) was crushed into powder. The powder was immersed with absolute alcohol and incubated at room temperature until the reach of saturation of extracted material. (There was no change in OD 363 with longer incubation). The alcohol extract (AE) was diluted 3 folds to ten folds in distilled water. The mixture immediately turned cloudy and GMD 1630 was found in precipitate after centrifugation at 5000 X g for 10 minutes. It was found possible to collect the purified GMD 1630 with repeated solubilization and precipitation between alcohol and water solvent systems. In practice, 20-30 gm G.M.D. resin block was extracted in 300-500 ml of absolute alcohol at $20^\circ$C-$75^\circ$C for 4-6 hours. Debris was removed and solution was centrifuged at 5000xg to eliminate any particle. For re-precipitating the active component from absolute alcohol, distilled water was added to dilute the 100% alcohol to 30-40%. Solution immediately turned turbid and active component GMD 1630 were collected in the pellet after centrifugation.

For chloroform extraction, GMD resin was first dissolved in distilled water. In regular practice, 20-30 gm was dissolved in 300-500 ml distilled water at 20-75° C for 30 minutes with continuous shaking. The solution was then extracted with chloroform/after partition system (equal volume on both phases). Extraction was performed until most of the yellowish component went from upper aqueous phase to lower chloroform phase. The lower phase was carefully collected. The upper phase was further extracted with the identical procedure to obtain more the active component. Both lower phases of two extractions were pooled and concentrated by chloroform evaporation with gas blowing or heating.

In our experiment, it is obvious that the purification of component GMD 1630 depends on both alcohol extraction and chloroform extraction. The order of procedures does not affect the result of purification; however the number of extractions does. In regular preparation, four extraction procedures were performed: two with chloroform extraction and two with alcohol extraction.

Routinely the final product was tested for its cytolytic property on cultured CE cells. Also the product was studied by paper chromatography and infrared spectroscopy for its purification. characteristically, sharp IR absorbance peaks were found at wavelengths 1740, 1690, 1630, 1590, 1430, 1380, 1340, 1260, 1180, 1140 and 1045 (1/cm). (1630-1590-1430-1140 for the four major peaks ranged from the highest)

In the drawings:

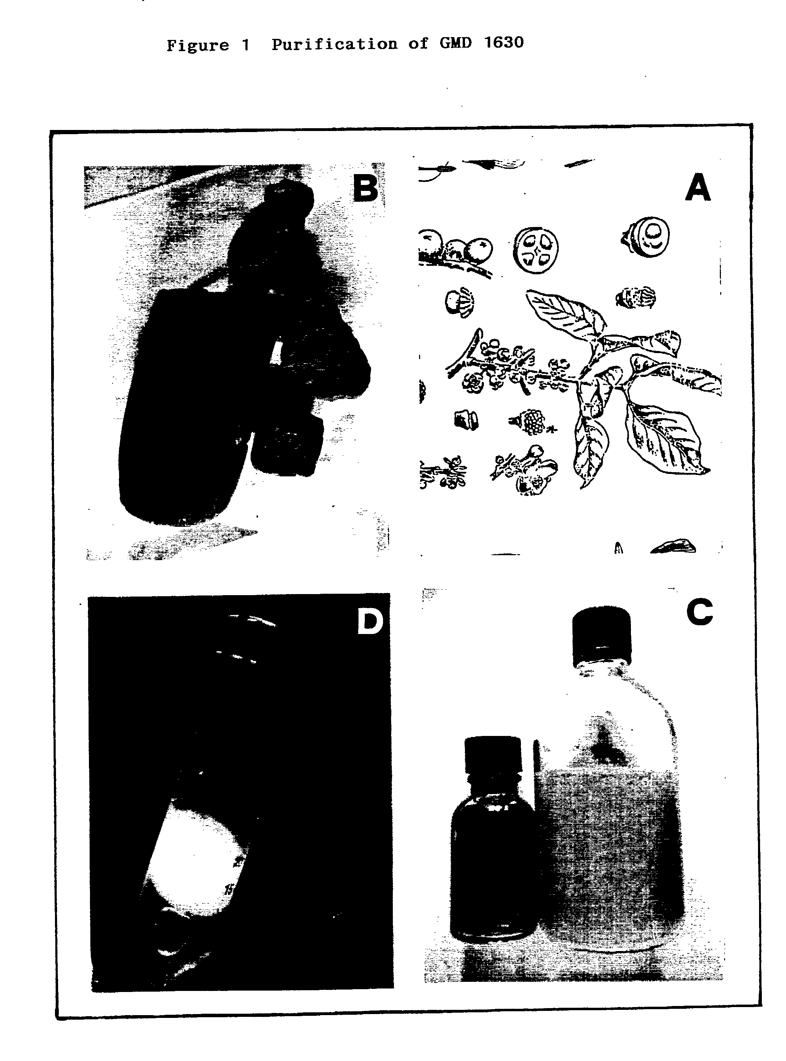

Fig. 1 Purification of GMD 1630.

A. Plant Garcinia morella Desv.

B. Resin Blocks.

C. Left: small bottle of 85% alcohol solution with GMD 1630.

Right: big bottle of GMD 1630 in aqueous suspension.

D. Chloroform/water partition system. In upper aqueous unwanted components were discarded; while in lower chloroform phase GMD 1630 was fully dissolved.

Fig. 2 Biological Effect of Purified GMD 1630 on CE Sarcoma Cells.

A. Control group (no GMD 1630 added): CE sarcoma cells at early confluent stage fixed and stained with amidoblack.

B. Experiment group (GMD 1630 added): Cell lysis was observed.

C. CE cells of A. observed under light microscopy (100 X).

D. CE cells of B. observed under light microscopy (100 X).

Fig. 3 Thin-layer Chromatography of GMD 1630 Preparation.

Five peaks were identified with solvent system chloroform/methanol/acetic acid (40:40:20 by volume).

Fig. 4 Infrared Absorbance Spectroscopy.

Both crude (dot line) and purified (solid line) GMD 1630 preparations were pressed into a KBr thin disc for IR scanning. The scan was performed with a Nicolet Fourier-Transform IR Spectrophotometer. The background of pure KBr was routinely substracted via computer. Data indicated major IR absorbance peaks at wavenumber (1 cm) 1140, 1180, 1260, 1340, 1380. 1430, 1590, 1630 (highest), 1690 and 1740.

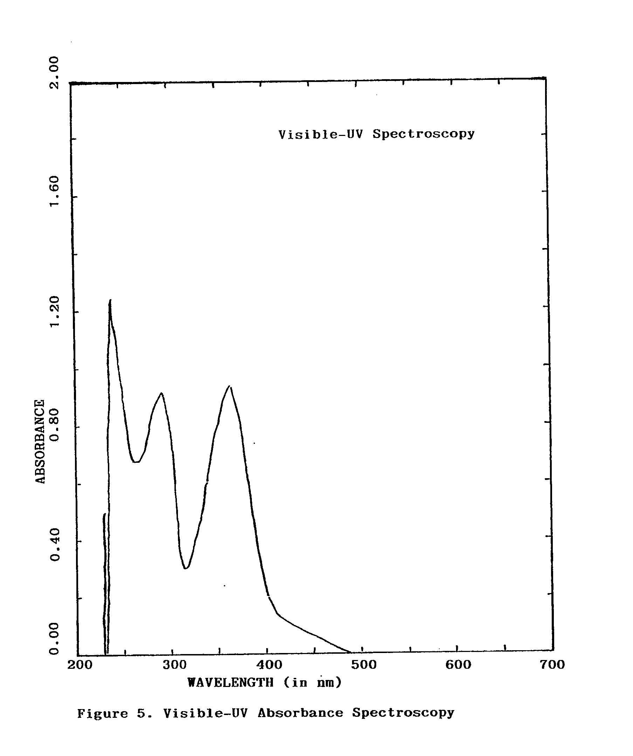

Fig. 5 Visible-UV Absorbance Spectroscopy.

For visible-UV spectroscopy, GMD 1350 was dissolved in 100% chloroform 100% and scanned with a Beckman DU-64. Result indicated two major absorbance peaks in region 250nm-400nm.

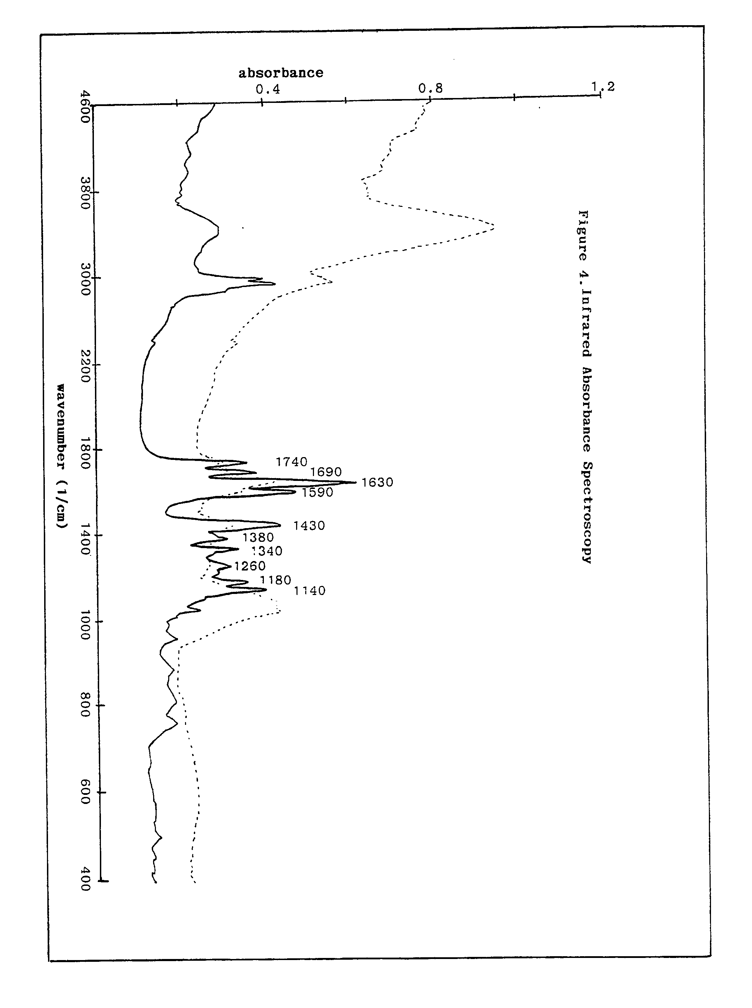

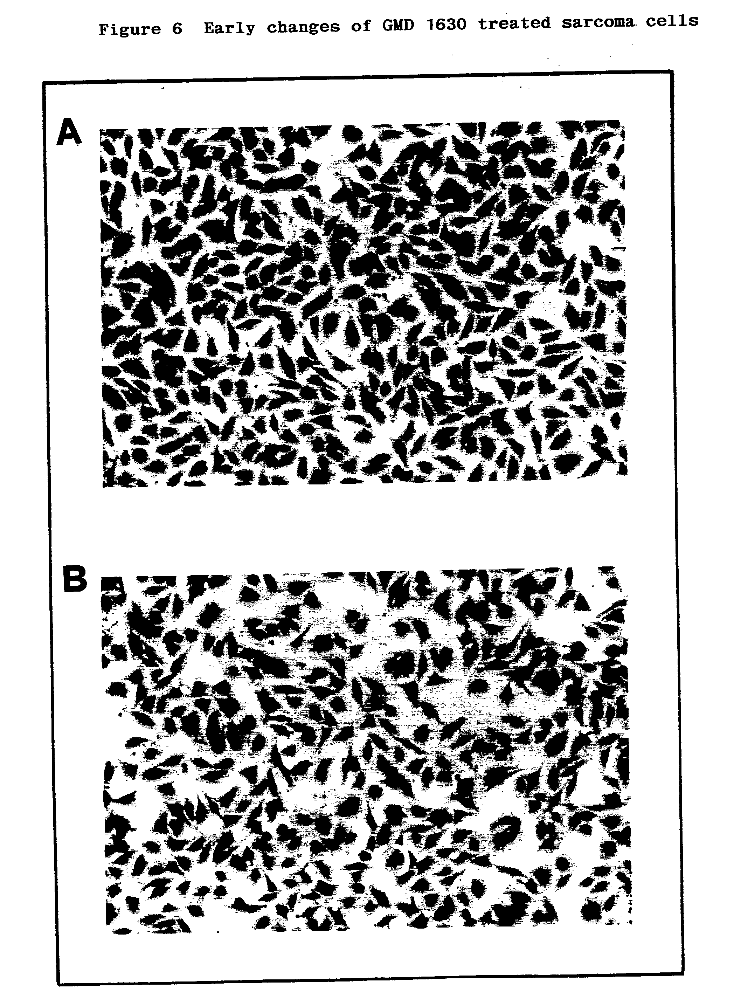

Fig. 6 Early Changes of GMD-1630 Treated Sarcoma Cells.

CE cells (sarcoma cell line) were treated with purified GMD 1630 (B) at 37° C for 20 minutes and stained for comparison with untreated cells (A). Affected cells marked by ( ) signal show characteristic "club cell" appearance.

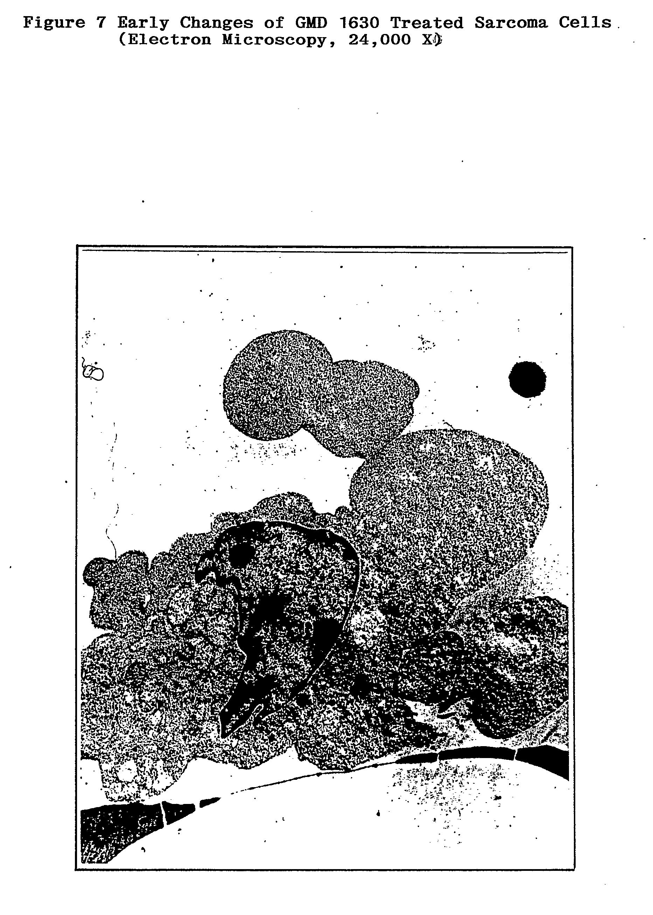

Fig. 7 Early Changes of GMD-1630 Treated Sarcoma Cells (Electron Microscopy, 24,000 X).

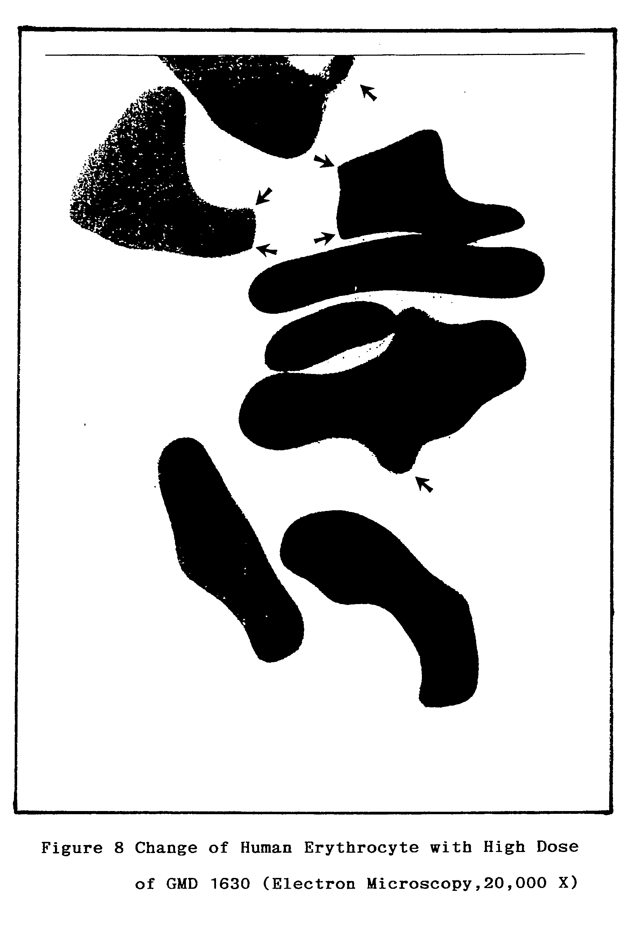

Fig. 8 Change of Human Erythrocyte with High Dose of GMD 1630 (Electron Microscopy, 20,000 X).

Human red blood cells were treated with high concentration (50 folds the dose for CE cells) at 37° C for 30 minutes. Some red blood cells lose the normal shape and turn square (arrows).

Fig. 9 Toxicity Test (EKG).

Balb/c mice were under general anesthetic condition and observed for any EKG change after injection of dissolved GMD 1630 (indicated by a "*" sign).

There is no significant change found within two-hour or longer period of observation.


EXAMPLES

3

The following examples are intended to illustrate specific embodiments of the invention. They are not intended to limit the invention in any manner.

I. Purification of GMD 1630 from GMD Resin.

Materials and Methods.

Materials - Dulbecco-Vogt modification of Eagle's medium, fetal bovine serum, trypsin-EDTA solution, penicillin/streptomycin solution and Ham's F12 medium were from Gibco (Grand Island, New York, U.S.A.). L-glutamine, phosphate were obtained from Sigma (St. Louis, MO, U.S.A.). Ethanol and chloroform were from Fisher (Medford, MA).

a. Preparation of Purified GMD 1630 from GMD Plant Fluid Collection of GMD Resin fluid -

Stem of plant GMD was cut and wound inserted with a tube for drainage. The collected fluid was heated to 75°C and evaporated to complete dryness. The dried plant resin showed a yellowish waxy appearance on its cut surface.

b. Purification of GMD 1630 from GMD Resin (figure 1) -

GMD resin was extracted for the active cytolytic component by various combinations of two extraction procedures: One by alcohol extraction and the other by chloroform extraction. For alcohol extraction, resin was first crushed and milled into powder. Twenty to thirty grams of GMD powder were dissolved in 200 to 300 ml 100% absolute alcohol with vigorous stirring at 20-75°C. In practice complete dissolution was observed overnight with saturation of absorbance at 360nm. To concentrate GMD 1630 dissolved in alcohol, the solution was diluted with distilled water to 20% alcohol or less and chilled on ice. Cloudy precipitate was immediately seen and GMD 1630 in precipitate was concentrated by a quick spin.

Thin-Layer chromatography.

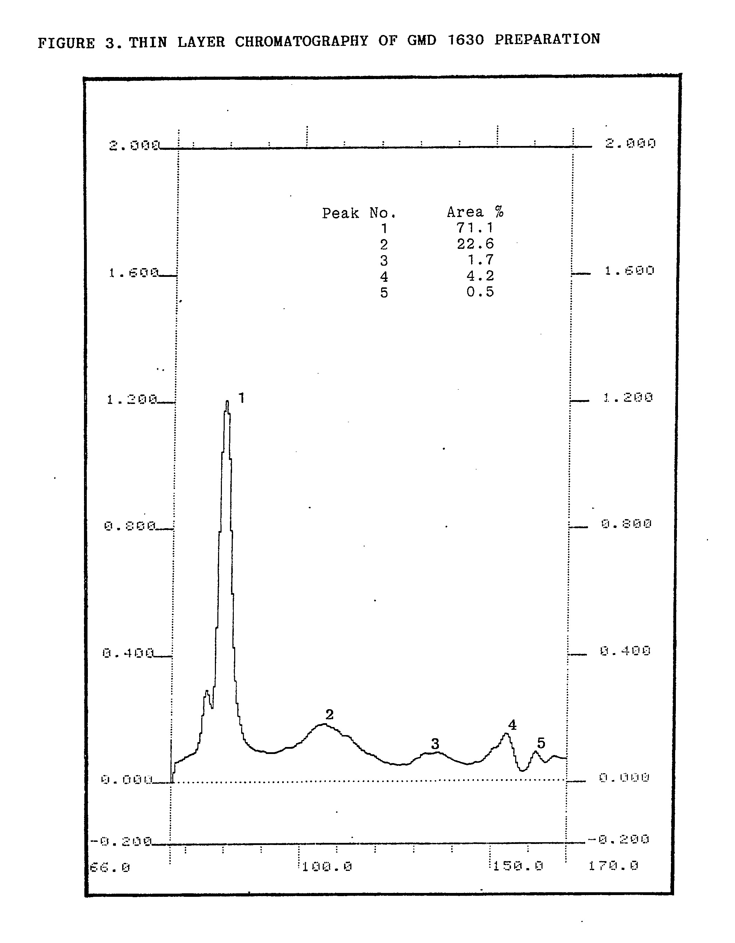

Final purification and analysis of GMD 1350 was performed by the use of thin layer chromatography. Concentrated GMD 1630 containing preparation in chloroform was obtained by evaporation. The solvent system used for thin-layer chromatography was chloroform/methanol/acetic acid (40:40:20 by volume) (figure 3). The components were identified with iodine vapor, or sulfuric acid charring. For preparing the best purified GMD 1630, spot of yellow component was removed by scratching the powder off and redissolve in chloroform.

c. Infrared Absorbance Spectroscopy and Visible-UV absorbance Spectroscopy.

Purified GMD 1630 in 80% ethanol was lyophilized to complete dryness and treated as solid sample for IR spectroscopy. Dried GMD 1630 were well-mixed with 100 mg of carefully dried spectroscopic grade KBr in a mullite mortar. The resulting mixture is placed in a stainless steel die under vacuum pump. The sample was then pressed in a hydraulic press at a pressure of about 4500kg/cm square for 5 minutes. The piston and the base of the press are then removed, and the pellet film was placed in the IR sample holder for direct observation. The scanning (figure 4) was performed from 400 to 4800 wavenumber with a Nicolet Fourier-Transform IR Spectrophotometer. The background of pure KBr was routinely subtracted from the data of GMD 1630-KBr pellet via computer.
For visible-UV spectroscopy, GMD 1350 was dissolved in chloroform or 100% ethanol and scanned from wavelength 200nm to 700nm with a Beckman DU-64 (figure 5).

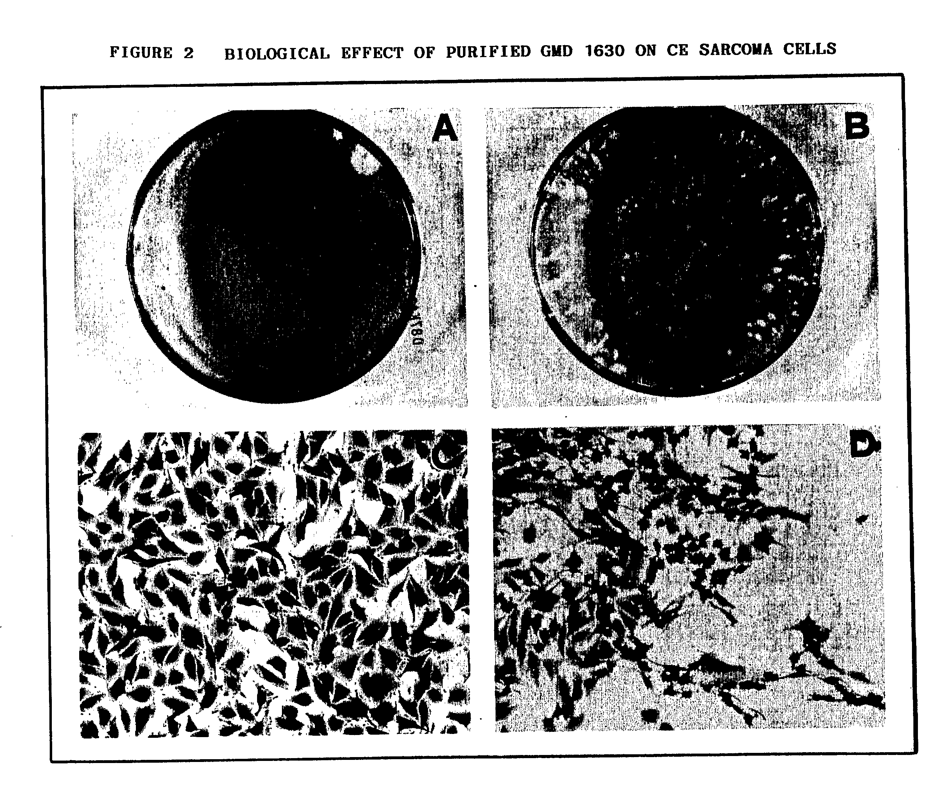

II. In Vitro Application of GMD 1630 as a Potent Cytolytic Agent (figure 2).
a. Culture of CE Cells from Endometrial Tumor for Cytolytic Assays (figure 6).

a. CE cells dissociated from an endometrial tumor specimen by collagenase I were grown in DMEM plus 10% FCS medium to their early confluent stage. Medium was freshly replaced every other days. Cells were incubated at 37°C culture incubator with humidified 5% CO2/95% air. CE cells became in vitro conditioned as an established cell line after 65 population passages. For cytolytic assay, CE cells were grown to their early confluent stage and added with purified GMD 1630. In most of our experiments, 10ul of Purified GMD 1630 dissolved in 50% alcohol (OD 360 = 250) was added in 10ml culture plate of CE cells at early confluent stage; while 10 ul of 50% alcohol was given as the control. Lysed cells were washed away with isotonic phosphate buffer and plates were fixed for amidoblack stain after 4 hours of incubation. Cells of both groups were further examined by light microscope. Degree of cell lysis was evaluated by measuring the loss of Amidoblack stain (compared with control group without addition of GMD 1630).

b. Electron Microscopy of Cultured CE Cells and Red Blood Cells Treated with GMD 1630 (figure 7 and 8).

Treated CE cells (figure 7) which became floating in growth medium were collected by centrifugation. For electron microscopy, these cultures were washed, fixed in 2% glutaraldehyde, postfixed in 1% osmium tetroxide, and prestained in 1% uranyl acetate. The cultures were then embedded in araldite-epon and cut in 500 A sections. The sections were stained with 2% uranyl acetate and Reynold's lead, and examined in Zeiss microscope. Red blood cells (figure 8) were also studied by E.M. for examining the fine changes.

III. In Vivo Application of GMD 1630 as a Potent Anticancer Agent.

TMC 97 hybridoma was established for other purpose and appeared an excellent cell line for ascites formation. A431 is a gifted cell line from California U.S.A. and develops tumor in nude mice. Anticancer effect of GMD 1630 was evaluated with these two cell lines and animal models.

Table 1 and 2 in Vivo Study of Anticancer Effect of GMD 1630.

Table 1.

| Effect of GMD 1630 on TMC 97 hybridoma of Balb/c Mice. | | | | | | |
|---|---|---|---|---|---|---|
| Injected Material | Mice | Dosage (ug/g) | Examined Parameters (mean/SD) | | | |
| | | | WBC x10 | RBC x10 | HCT % | Ascites ml |
| | | | (in nm) | | | |
| Control (saline) | 50 | | 4.6/0.5 | 8.0/0.6 | 38/2 | 20/2 |
| GMD 1630 | 50 | 2.75 | 4.9/0.7 | 8.4/0.5 | 42/3 | 7/1 |
| GMD 1630 | 50 | 1.25 | 4.8/0.2 | 7.9/0.3 | 40/3 | 11/2 |
| GMD 1630 | 50 | 0.25 | 4.5/0.2 | 8.2/0.3 | 42/2 | 15/3 |

One million TMC 97 hybridoma cells were injected into peritoneal cavity of each Balb/c mouse for ascites.
Animals were killed twenty days after and amount of ascites in each animal was measured. WBC, RBC and HCT were also measured and compared.

Table 2.

| Effect of GMD 1630 on Tumor Growth of Human Cervical Carcinoma A431 in Nude Mice | | |
|---|---|---|
| Nude Mice (No.) | Injected Material (ug/g) | Tumor Size mm x mm |
| 1 | Saline | 20x30 |
| 2 | Saline | 18x28 |
| 3 | Saline | 22x32 |
| 4 | GMD 1630 (3,0) | 4x 8 |
| 5 | GMD 1630 (3,0) | 5x 7 |
| 6 | GMD 1630 (2,0) | 8x11 |
| 7 | GMD 1630 (1,5) | 7x10 |
| 8 | GMD 1630 (0,5) | 15x18 |
| 9 | GMD 1630 (0,5) | 18x20 |
| 10 | GMD 1630 (0,2) | 20x25 |

One million cells of A431 (human cervical carcinoma-epidermoid type) were injected subcutaneously into one site of an adult nude mice. GMD 1630 of described dosage was given intramuscularly twice a week since the day of tumor cell injection. Size of tumor was measured three weeks after.

Toxicity tests were further performed with blood pressure, heart rate, EKG (figure 9), blood clotting and allergy tests. results indicated that there were no significant difference between control and experiment groups. Biochemical data indicated that animal treated with GMD 1630 was not much different from untreated animal (table 3).

Table 3

| Biochemical Analysis of Treated and Untreated Animals | | |
|---|---|---|
| | Control | Treated |
| Albumin | 0.4 mg% | 0.4 mg% |
| Globulin | 3.7 mg% | 3.6 mg% |
| Total Protein | 4.1 mg% | 4.0 mg% |
| SGOT | 7 mu/ml | 8.0 mu/ml |
| SGPT | 4 mu/ml | 5 mu/ml |
| Alkaline Phosphatase | 14 mu/ml | 18 mu/ml |
| Total Bilirubin | 0 mg% | 0 mg% |
| BUN (Blood Urea Nitrogen) | 12 mg% | 13 mg% |
| Creatinine | 0.5 mg% | 0.3 mg% |
| Calcium | 6.5 mg% | 6.5 mg% |
| Phosphate | 3.6 mg% | 3.5 mg% |
| Uric Acid | 0/5 mg% | 1.0 mg% |
| Total Cholesterol | 75 u/ml | 61 u/ml |
| Na | 138 nM | 140 mM |
| K | 6.8 mM | 7.6 mM |
| Cl | 98 mM | 93 mM |

In treated animal group, animals Balb/c mice were injected as described in table 1 with dose 2.0ug/gm every other day for two months. Biochemical data was obtained with a Hitachi Automatic Analyser model 736-740 (Japan).

# EP 0 428 815 A1

**Claims**

1. A method for purifying a cytolytic compound GMD 1630.

2. The method of claim 1 wherein the other components without cytolytic activity is substantially removed.

3. The method of claim 1 wherein the active component is extracted with alcohol solubilization process and concentrated with a rapid precipitation process by diluting the alcohol solution into water.

4. The method of claim 1 wherein the active component is extracted with chloroform/water partition system.

5. The method of claim 1 wherein the active component is chemically identified as the substance giving IR absorbance peaks at wavelengths (1/cm) 1630, 1590, 1430 and 1140, as well as the method of quick chemical identification with characteristic visible and UV absorbance peaks at wavelengths (nm) 290 and 360 (with a deep valley at 315 nm wherein the ratio of absorbance values A 290 : A 315 : A 360 is 2:1:2).

6. The method of claim 1 wherein the attached mammalian cells are used for direct biological assays as described in figures 2 and 6, attach cells being fixed and stained in situ with amidoblack, the degree of cell lysis being measured as the decreased stained cells on plates with added toxin.

7. An original method which makes cell lysis in isotonic or hypertonic solutions by adding GMD 1630, the method involving severe morphological changes which are due to the binding of GMD 1630 to intracellular structure, possibly cytoskeleton.

8. An application of GMD 1630 for anticancer treatment, wherein it exhibits advantages over other conventional drugs such as low acute and subacute toxicity, lack of surpression on blood cell generating system and lack of malfunction with many other systems such as gastrointestinal, hepatic and renal systems.

Figure 1  Purification of GMD 1630

FIGURE 2   BIOLOGICAL EFFECT OF PURIFIED GMD 1630 ON CE SARCOMA CELLS

FIGURE 3. THIN LAYER CHROMATOGRAPHY OF GMD 1630 PREPARATION

Figure 5. Visible-UV Absorbance Spectroscopy

Figure 4. Infrared Absorbance Spectroscopy

Figure 6   Early changes of GMD 1630 treated sarcoma cells

Figure 7 Early Changes of GMD 1630 Treated Sarcoma Cells
(Electron Microscopy, 24,000 X)

Figure 8 Change of Human Erythrocyte with High Dose of GMD 1630 (Electron Microscopy,20,000 X)

Figure 9 Toxicity Test (EKG)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 360 311 (UNIVERSITY OF CHICAGO) * Page 9, line 8 - page 10, line 33; claims 1-3 * | 1 | C 07 G 17/00<br>A 61 K 35/78 |
| A | CHEMICAL ABSTRACTS, vol. 67, no. 13, 25th September 1967, pages 5889-5890, abstract no. 62858c, Columbus, Ohio, US; K.V. NAGESWARA et al.: "Toxicity and pharmacodynamic effects of alfa-guttiferin",&INDIAN J. EXP. BIOL. 5(2), 96-100(1967) * Abstract * | 1 | |
| A | CHEMICAL ABSTRACTS, vol. 67, no. 13, 25th September 1967, page 5890, abstract no. 62859d, Columbus, Ohio, US; K.V. NAGESWARA et al.: "Antibiotic principles of Garcinia morella. IX. Antimicrobial activity of alpha- and beta-guttiferins and their derivatives", & INDIAN J. EXP. BIOL. 5(2), 101-5(1967) * Abstract * | 1 | **TECHNICAL FIELDS SEARCHED (Int. Cl.5)**<br><br>C 07 G 17/00<br>A 61 K 35/00 |
| A | CHEMICAL ABSTRACTS, vol. 69, no. 23, 2nd December 1968, page 8871, abstract no. 94896w, Columbus, Ohio, US; K. SANTHANAM et al.: "Antibiotic principles of Garcinia morella. XII. Characterization of beta- and alphal-guttiferins as cathartic principles of gamboge and seed coat of G. morella", & INDIAN J. EXP. BIOL. 1968, 6(3), 158-9 * Abstract *<br><br>                    -/- | 1 | |

The present search report has been drawn up for all claims

| Place of search | Date of completion of the search | Examiner |
|---|---|---|
| THE HAGUE | 11-07-1990 | BRENNAN J. |

**EUROPEAN SEARCH REPORT**

Application Number

EP 89 83 0521

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | TETRAHEDRON LETTERS, no. 7, 1963, pages 459-472, Pergamon Press Ltd, GB; G. KARTHA et al.: "The constitution of morellin" <br> * Page 462, line 5 - page 465, line 5 * | 1 | |
| A | TETRAHEDRON LETTERS, no. 14, 1974, pages 1259-1262, Pergamon Press, GB; G.S.R. SUBBA RAO et al.: "The structure of moreollin" <br> * Page 1259 * | 1 | |

TECHNICAL FIELDS
SEARCHED (Int. Cl.5)

The present search report has been drawn up for all claims

| Place of search | Date of completion of the search | Examiner |
|---|---|---|
| THE HAGUE | 11-07-1990 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 01.82 (P0401)